Europäisches Patentamt

**European Patent Office**

**Office européen des brevets**

(11) Publication number: **0 193 683**
A1

# (12) EUROPEAN PATENT APPLICATION

(21) Application number: **85309516.4**

(22) Date of filing: **30.12.85**

(51) Int. Cl.⁴: **G 01 N 27/00,** G 01 N 30/00

(30) Priority: **05.01.85 GB 8500280**
**07.09.85 GB 8522264**

(43) Date of publication of application: **10.09.86**
**Bulletin 86/37**

(84) Designated Contracting States: **AT DE FR GB IT**

(71) Applicant: **Lion Laboratories Limited, Ty Verlon Industrial Estate, Barry South Glamorgan, CF6 3BE (GB)**

(72) Inventor: **Jones, Thomas Barry, 20 South Road Sully, Near Cardiff Gwent (GB)**

(74) Representative: **Dunlop, Brian Kenneth Charles et al, c/o Wynne-Jones, Lainé & James Morgan Arcade Chambers 33 St. Mary Street, Cardiff, CF1 2AB (GB)**

(54) **Method and apparatus for determining the concentration of components of liquid systems.**

(57) This invention relates to methods and apparatus for determining the concentration of volatile oxidisable components of liquid systems, and in particular to the measurement of ethanol concentration of alcoholic beverages.

The apparatus 10 has a pair of head-space vessels 14, 15 for receiving a dilute sample of the beverage and of an ethanol standard respectively. The air pump 23 passes air through first the standard and subsequently the dilute beverage. In each event, when equilibrium has occurred, a sample of the gas/vapour mixture in the head-space of the vessel concerned is passed to a fuel cell 33 which measures the ethanol concentration of the liquid. The measurement made from the standard is used to calibrate the fuel cell 33 and the output signal of the fuel cell 33 can be weighted in accordance with the expected concentration of the sample beverage. The apparatus is portable and simple to use.

- 1 -

## Method and Apparatus for Determining the
## Concentration of Components of Liquid Systems

This invention relates to methods and apparatus for determining the concentration of volatile oxidisable components of liquid systems.

In many industries, and in the brewing industry in particular, there is a need to be able to determine the concentration of volatile oxidisable components of liquid systems, e.g. ethanol concentrations. There are of course similar applications in the wine and spirits industries and other fields and include the manufacture of yeast, fruit storage and soft drinks. Until now the principal methods of detecting such components have been distillation, hydrometers and gas liquid chromatography. In addition in certain fields such as breath alcohol monitoring, fuel cells have been used. In practice there are many difficulties with the respective methods, some take a long time and others require very sophisticated equipment and technical skill.

It has also been proposed to measure such components by static head-space analysis, but this requires considerable technical skill and time.

It is an object of this invention to provide a method and/or apparatus for measuring volatile oxidisable components of a liquid system which can be utilised in a short time and with little training.

From one aspect the invention consists in a method of detecting or measuring the presence of a volatile oxidisable component of a liquid system, comprising causing gas to flow through or in contact with a dilute solution of the liquid system, and contacting the resulting gas vapour mixture or a sample hereof with an electrical gas sensor to obtain an indication or measurement of the component concentration in the liquid system.

Preferably the sample is contacted with the gas sensor, which may be an electrochemical fuel cell, when equilibration has occurred.

In a preferred arrangement the method includes calibrating the gas sensor by obtaining an indication of the component concentration of the component standard by the method as set out above. In this case the method may further include measuring the temperature difference between the standard and the solution and adjusting the calibration in accordance with that temperature difference.

Conveniently the gas may be bubbled through the liquid system, in which case the bubbles may have a temperature between 0.25 mm and 0.75 mm and may be passed through the gas for between 1½ to 3 minutes at a rate of 250 to 350 $cm^3$ per minute.

In order to maintain the linearity of the gas sensor the degree of dilution of the liquid system may be varied in accordance with its expected concentration and the output of the gas sensor may be amplified to compensate for the degree of dilution. For example, if the component is ethanol and the volume of the dilute liquid solution is 100 $cm^3$, then the volume of the liquid system in the solution is 2 $cm^3$ if the expected concentration is 5% v/v; 1 $cm^3$ for 10% v/v; 0.5 $cm^3$ for 20% v/v and 0.25 $cm^3$ for 40% v/v or pro rata accordingly.

Particularly for such effervescent liquid systems as beer, it is preferred that an anti-foaming agent is added to the solution before gas is passed through the solution.

From another aspect the invention consists in apparatus for use in detecting or measuring the presence of a volatile oxidisable component of a liquid system, comprising a liquid sampling chamber for receiving a dilute sample of the liquid system, means for passing gas through or in contact with the liquid system to emerge into a gas space in contact with the liquid system and means including an electrical gas sensor for detecting or measuring the component concentration in the emergent gas vapour mixture.

The gas space may at least in part be constituted by a head-space defined by the liquid sampling chamber and the means for passing gas through the liquid system may include an air pump for causing the gas to bubble through the dilute sample.

Preferably the apparatus further comprises a second chamber for a component standard, means for passing gas through or in contact with standard to emerge into a gas space in contact with a liquid system and means for feeding at least a sample of the emergent gas to the electrical gas sensor and means for calibrating the gas sensor in accordance with the component concentration measured or detected from the standard.

The apparatus may further include means for detecting the temperature difference between the liquids in the chambers and for compensating the output of the gas sensor accordingly. Additionally or alternatively the apparatus may include a variable gain amplifier for amplifying the output of the sensor and means for altering the gain in accordance with the degree of dilution of the liquid system in the solution.

The apparatus may include means for automatically cycling the procedure to obtain a number of indications of the component concentration in the liquid system, in which case means may be provided for displaying the mean of the set of the indications.

The apparatus may be portable and may include an air pump, motorised valves and an electrical fuel source.

Although the invention has been defined above

0193683

- 5 -

it is to be understood that it includes any inventive combination of the features set out above or in the following description.

The invention may be performed in various ways and a specific embodiment will now be described with reference to the accompanying drawings, in which:

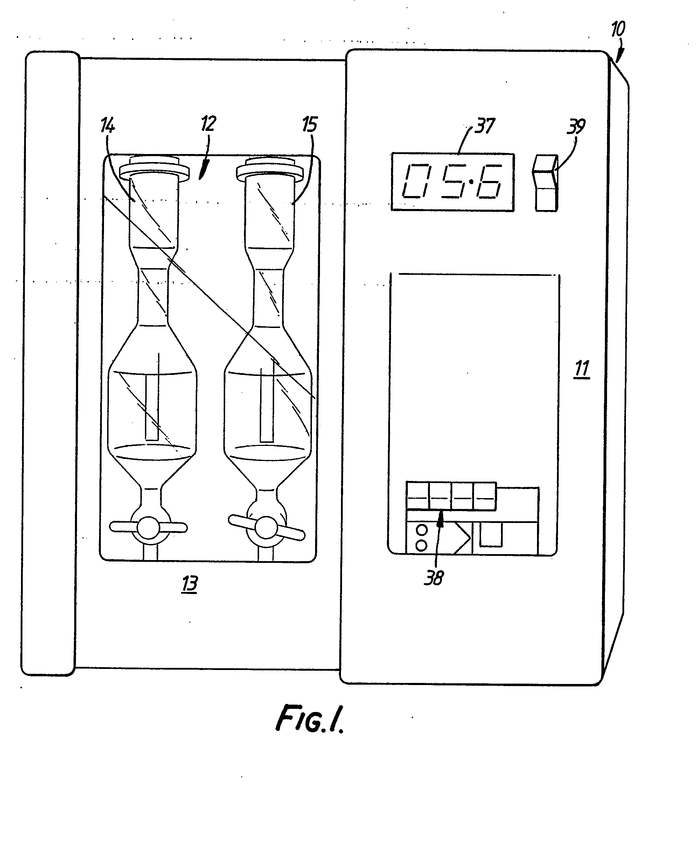

Figure 1 shows apparatus for determining the concentration of a volatile oxidisable component of a liquid system;

Figure 2 is a schematic enlarged view of a head space generating vessel for use in the apparatus; and

Figure 3 is a schematic flow diagram of the apparatus.

Although, as has been mentioned above, the apparatus may have many uses, for the purposes of simplicity it will be described in connection with the detection of ethanol in alcoholic beverages.

It is known that if you place a sample of a liquid system in a vessel having a gaseous space above it, then volatile substances will evaporate into the gas and eventually an equilibrium will be achieved. In this state the concentration in the liquid of the volatile component is directly proportional to the concentration in the gas. It has now been discovered that if a gas is bubbled through the liquid system, the system

BKCD/SMR                    - 5 -

very rapidly reaches a state of equilibrium and therefore surprisingly this simple technique enables head-space measurements to be taken extremely rapidly. Turning to the Figures, an apparatus for determining the ethanol concentration of a dilute sample of an alcoholic beverage is generally indicated at 10. The apparatus comprises a casing 11 which defines a chamber 12 closed by a door 13. Within the chamber 12 are mounted head-space vessels 14, 15 which are illustrated in more detail in Figure 2. The vessels are in fact identical and so for convenience only vessel 14 will be described.

As can be seen from Figure 2, the vessel 14 has a bulbous reservoir portion 16 for receiving a dilute liquid sample, which can be removed after testing through a valve liquid outlet 17. A tube 18 extends into the reservoir 16 with its outlet 19 close to the bottom thereof. This tube provides an air inlet and also allows a thermocouple to be introduced into the liquid. Above the reservoir extends a neck portion 20 which is closed by a plastic stopper at its upper end and has a lateral air outlet 21. A calibration mark 22 is scored on the neck 20 and represents a volume of 100 $cm^3$ when the reservoir is filled up to this point.

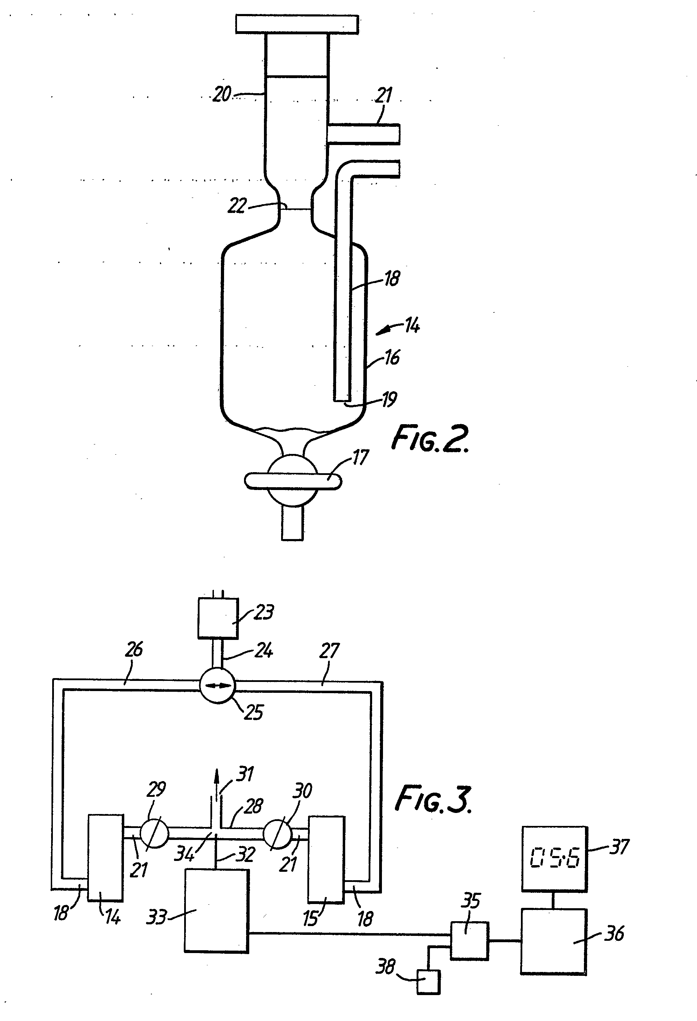

The operational arrangement of the apparatus can best be seen in Figure 3. Here an air pump 23 supplies air at the rate of approximately 300 $cm^3$ per minute to an air inlet 24. This inlet is valved by a two-way tap

25 which passes the air to either a branch 26 or a branch 27. The first is connected to the air inlet tube 18 of vessel 14 and the second to the air inlet tube of the vessel 15. Each air outlet 21 of the vessels 14 and 15 is connected to a T-piece 28 via respective valves 29, 30. The leg of the T forms an air outlet 31 and the sampling needle 32 of an electrochemical fuel cell 33 is disposed in the intersection of the T at 34.

The output of the fuel cell 33 is passed through an amplifier 35 and through processing and compensating circuitry 36 to a digital display 37. The gain of the amplifier is adjustable by means of a multiple switch 38. This adjustment is desirable because it is difficult to provide fuel cells which operate linearly over a wide range of concentrations. It is therefore preferred to adjust the dilution of samples in accordance with their expected ethanol concentration and to adjust the gain to compensate for this.

In use a volume of ethanol standard is pipetted into water within the vessel 14, the vessel having first been thoroughly cleansed, and the volume of liquid in that vessel is then made up with water to the calibration mark 22. The same volume of the beverage to be tested, say beer, is introduced into the vessel 15 and similarly diluted. The air pump 23 is then started by on/off switch 39 and tap 25 is positioned to pass the air along branch 26 to bubble through the ethanol standard quickly establishing equilibrium. It has been found that equilibrium exists after about two minutes and when this occurs the valve 29 is opened and a sample of the extracted head-space gas is taken by the fuel cell 33 as the gas passes through the T-piece 28. The measured concentration is detected by the circuitry 36 and compared with the preset value of the standard. If there is any divergence the fuel cell output is compensated. The tap 25 is then repositioned to pass air through the vessel 15, after a short cycle time for purging the T-piece 28. The same procedure is followed and the fuel cell obtains a sample of the head-space gas from vessel 15. The output of the fuel cell 33 is then processed and displayed digitally at 37.

It is preferred that another calibration cycle is then carried out to check that the fuel cell is still correctly compensated and if it is not the apparatus will indicate a false reading.

The head-space concentration is extremely temperature susceptible and accordingly the temperature of the liquid samples is measured by means of thermocouples 40 and output signal of the fuel cell 33 is suitably compensated by circuitry 36 in accordance with the temperature difference. Alternatively the vessels may be maintained at a constant temperature by use of a temperature controlled heater.

If a wine is now to be tested, exactly the same procedure is followed except the switch 34 is placed on a different range setting because of the higher alcoholic concentration of wine. Conversely, a smaller sample of wine is placed in the vessel 15. Typical figures for the dilution with respect to anticipated approximate concentration are indicated above.

It will be appreciated that the apparatus is extremely portable and can provide rapid accurate concentration readings in any location without sophisticated technical skills being required. For example, it can be taken on horse back to the outlying farms in Portugal where port is produced and which until now have had hardly any scientific quality control available to them.

The basis of the invention and the measurement techniques involved is that there is a constant ratio between the concentration of alcohol in the vapour above the solution and the concentration of alcohol in the solution: this is known as Henry's Law. The so-called partition ratio depends on temperature amongst other factors and hence the need for accurate temperature control or measurement and compensation.

The partition ratio is also affected by other substances present in the fermenting liquor, i.e. sugar and other dissolved species. However, when the liquor is diluted, say one hundred times, these effects are negligible. The high sensitivity of the fuel cell sensor makes it possible by this method to measure to very low concentrations of alcohol and be unaffected by these substances present.

The invention can also be applied to a test instrument and technique for continuous measurement of alcohol levels in a liquid, during processes such as fermentation or inter-mixing with other liquids, by incorporating a system for accurate automatic dilutions of a continuous or semi-continuous sample and passing the diluted solution then continuously or semi-continuously to the analysis vessel.

## CLAIMS

1. A method of detecting or measuring the presence of a volatile oxidisable component of a liquid system, comprising causing gas to flow through or in contact with a dilute solution of the liquid system, and contacting the resulting gas vapour mixture or a sample hereof with an electrical gas sensor to obtain an indication or measurement of the component concentration in the liquid system.

2. A method as claimed in claim 1 wherein the sample is contacted with the gas sensor when equilibration has occurred.

3. A method as claimed in claim 1 or 2 of the preceding claims including calibrating the gas sensor by obtaining an indication of the component concentration of a component standard by the method of any one claims 1 or 2.

4. A method as claimed in claim 3 further including measuring the temperature difference between the standard and the solution and adjusting the calibration in accordance with that temperature difference.

5. A method as claimed in any one of the preceding claims wherein the gas is bubbled through the liquid system.

6. A method as claimed in claim 5 wherein the bubbles have a diameter between 0.25mm and 0.75mm.

7.  A method as claimed in claim 5 or claim 6 wherein the gas is bubbled through for between 1½ to 3 minutes at a rate of between 250 to 350 $cm^3$ per minute.

8.  A method as claimed in any one of the preceding claims wherein the degree of dilution of the liquid system is varied in accordance with its expected concentration and the output of the gas sensor is amplified to compensate for the degree of dilution.

9.  A method as claimed in claim 8 wherein if the component is ethanol and the volume of the dilute liquid solution is 100 $cm^3$ then the volume of liquid system in the solution is 2 $cm^3$ if the expected concentration is 5% v/v; 1 $cm^3$ for 10% v/v; 0.5 $cm^3$ for 20% v/v and 0.25 $cm^3$ for 40% v/v or pro rata accordingly.

10.  A method as claimed in any one of the preceding claims including adding an anti-foaming agent to the solution before gas is passed through the solution.

11.  Apparatus for use in detecting or measuring the presence of a volatile oxidisable component of a liquid system, comprising a liquid sampling chamber for receiving a dilute sample of the liquid system, means for passing gas through or in contact with the liquid system to emerge into a gas space in contact with the liquid system and means including an electrical gas sensor for detecting or measuring the component concentration in the emergent gas vapour mixture.

12. Apparatus as claimed in claim 11 wherein the gas space is at least in part constituted by a head-space defined by the liquid sampling chamber.

13. Apparatus as claimed in claim 11 or claim 12 wherein the means for passing gas through the liquid system includes an air pump for causing the gas to bubble through the dilute sample.

14. Apparatus as claimed in any one of claims 11 to 13 wherein the apparatus further comprises a second chamber for a component standard, means for passing gas through or in contact with the standard to emerge into a gas space in contact with the liquid system and means for feeding at least a sample of the emergent gas to the electrical gas sensor and means for calibrating the gas sensor in accordance with the component concentration measured or detected from the standard.

15. Apparatus as claimed in claim 14 further including means for detecting the temperature difference between the liquids in the chambers and for compensating the output of the gas sensor accordingly.

16. Apparatus as claimed in any one of claims 11 to 15 further including a variable gain amplifier for amplifying the output of the sensor and means for altering the gain in accordance with the degree dilution of the liquid system in the solution.

17. Apparatus as claimed in any one of claims 11 to 16 further including means for automatically cycling the procedure to obtain a set of indications of the component concentration in the liquid system.

18. Apparatus as claimed in claim 17 further including means for displaying the mean of the set of indications.

19. Apparatus as claimed in any one of the preceding claims wherein the apparatus is portable and includes an air pump, motorised valves and an electrical fuel source.

0193683

FIG.I.

0193683

FIG.2.

FIG.3.

| | **DOCUMENTS CONSIDERED TO BE RELEVANT** | | EP 85309516.4 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | <u>DE - B2 - 1 773 510</u> (DYNAMIT)<br>* Claims; fig. 1,2 *<br>-- | 1,2 | G 01 N 27/00<br>G 01 N 30/00 |
| A | <u>GB - A - 1 582 117</u> (ISTITUTO)<br>* Claims; fig. *<br>-- | 1 | |
| A | <u>US - A - 3 319 159</u> (ROBINSON)<br>* Totality *<br>-- | 1 | |
| A | <u>EP - B1 - 0 045 554</u> (CHEMICAL)<br>* Page 2, lines 1-21; claims *<br>---- | 1,2 | |

| TECHNICAL FIELDS SEARCHED (Int. Cl.4) |
|---|
| G 01 N 27/00 |
| G 01 N 35/00 |
| G 01 N 37/00 |
| G 01 N 33/00 |
| G 01 N 30/00 |
| G 01 N 31/00 |

The present search report has been drawn up for all claims

| Place of search | Date of completion of the search | Examiner |
|---|---|---|
| VIENNA | 20-03-1986 | ERBER |